## Europäisches Patentamt

## European Patent Office

## Office européen des brevets

(11) Publication number: **0 214 527**

**B1**

# EUROPEAN PATENT SPECIFICATION

(45) Date of publication of patent specification: **30.01.91**

(51) Int. Cl.⁵: **A 61 F 11/04,** A 61 B 5/12, A 61 N 1/05

(21) Application number: **86111568.1**

(22) Date of filing: **21.08.86**

(54) External ear canal electrode to be placed proximate the tympanic membrane.

(30) Priority: **21.08.85 US 767324**

(43) Date of publication of application:
**18.03.87 Bulletin 87/12**

(45) Publication of the grant of the patent:
**30.01.91 Bulletin 91/05**

(84) Designated Contracting States:
**AT CH DE FR GB IT LI SE**

(56) References cited:
**EP-A-0 057 450**
**EP-A-0 084 972**
**EP-A-0 104 924**
**DD-A- 105 720**
**DE-A-3 006 472**
**US-A-3 531 992**

(73) Proprietor: **MINNESOTA MINING AND MANUFACTURING COMPANY**
**3M Center, P.O. Box 33427**
**St. Paul, Minnesota 55133-3427 (US)**

(72) Inventor: **Stypulkowski, Paul H. Minnesota Mining and**
**Manufacturing Co. 2501 Hudson Road P.O. Box 33427**
**St. Paul Minnesota 55133-3427 (US)**
Inventor: **Van den Honert, Christopher**
**Minnesota Mining and**
**Manufacturing Co. 2501 Hudson Road P.O. Box 33427**
**St. Paul Minnesota 55133-3427 (US)**

(74) Representative: **Baillie, Iain Cameron et al**
**c/o Ladas & Parry Isartorplatz 5**
**D-8000 München 2 (DE)**

Courier Press, Leamington Spa, England.

## Description

### Technical field

The present invention relates generally to electrodes for applying/recording electrical signals to/from the neural/neuromuscular system of a person. The present invention more particularly relates to such electrodes which are inserted into the external ear canal of a person.

### Background art

It is well known that electrical stimulation of the auditory system of a person can produce complex perceptions of sounds in human subjects. Experiments by Volta occurred in the early 1800's. Volta inserted metal bars into his external ear canals and passed current between the bars and reported the perception of sound. A series of studies was performed in the late 1930's by Jones, Stevens and Lurie which examined the relationship between various parameters of electrical stimulation and the listed auditory perceps. In these experiments, investigators used metal electrodes placed into saline filled external ear canals to deliver electrical current to the auditory system.

More recently, electrodes surgically implanted into the cochlea, are providing a sensation of hearing in profoundly deaf individuals. The development of cochlear implants has prompted significant additional research in the area of electrical stimulation for hearing augmentation.

Means of stimulating the auditory system which are noninvasive, as opposed to a cochlear implant, are desirable for several reasons. A noninvasive electrode system for auditory stimulation could be used as a functional auditory prosthesis, as a tool in the diagnostic evaluation of potential candidates for cochlear implants or to record the electrical signals generated by an auditory system stimulated by other means.

Several investigators have reported the use of electrodes combined with a saline filled ear canal (known as a Bremmer type electrode) to provide a noninvasive means of auditory stimulation. This approach to the stimulation of the auditory system has a major drawback. Current passed into the saline solution spreads rapidly in all directions through the tissues of the ear and head. The same electrical current which activates the auditory nerve can also stimulate cutaneous nerve fibers thereby producing uncomfortable and, possibly, painful sensations.

Other electrodes have been designed for use in the external ear canal. An example is the electrode disclosed in European Patent Application EP—A—0 084 972. This electrode has a rigid body with a compressible material mounted around the distal end of the body. The compressible material may carry an electrically conductive gel. The rigid body serves as an electrical conductor to connect the distal end of the electrode with the proximate end of the electrode. This electrode is not suitable for placement proximate the tympanic membrane or tympanomeatal annulus because the shape of the body does not permit deep insertion, because the compressible material is positioned radially with respect to the tip of the distal end of the electrode and, most importantly, because the rigid body would create extreme pain should the tympanic membrane be contacted during insertion or use.

Existing systems of auditory stimulation have significant disadvantages. Cochlear implants require surgical invasion of the body. Thus, cohlear implants are not practical for evaluation or diagnostic purposes, which involve temporary stimulation or recording. On the other hand, conventional external ear canal stimulation has significant limitations in dynamic range. The electrical current used in external ear canal stimulation must be above an amount to exceed the hearing threshold but must be below an amount which would produce pain within the external ear canal or, possibly, uncomfortable loud sensations.

### Disclosure of invention

The present invention solves these problems by eliminating surgical invasion while substantially increasing the dynamic range of the stimulation signals which can comfortably be utilized.

The only direct physical pathway from the external ear canal to the inner ear (aside from the skull itself) is the tympanic membrane and the ossicular chain. This tissue connection constitutes the major pathway for current flow between the inner ear and the external ear canal and visa versa. Electrical signals which are generated inside a normally functioning cochlea travel through surrounding tissues and can be recorded from outside of the cochlea. This technique is clinically known as electrocochleography (ECoG). The amplitude of ECoG signals is largest at the surface of the cochlea, the measurement of which is in invasive technique. For noninvasive measurements, the amplitude of ECoG signals is greatest at the tympanic membrane. This supports the hypothesis that the ossicles are the lowest resistance pathway by which current travels between the cochlea and the external ear canal. The present invention provides an electrode for stimulating or recording which takes advantage of this low resistance connection between the inner ear and the external ear canal. The electrode can be used to either stimulate the inner ear with electrical current or, conversely, to measure the electrical activity originating within the inner ear without an invasive technique.

According to this invention there is provided an electrode adapted to be utilized within the external ear canal for applying/recording electrical signals to/from the neural/neuromuscular system of a person having an external ear canal and adjacent tympanic membrane, having an elongate body having a proximate end and a distal end, having a compressible material mounted at said distal end of said body, having an electrically conductive gel carried by said compressible material, and having an electrical conductor coming into contact with said body, said electrical conductor being electrically coupled to said electrically conductive gel und adapted to be coupled to a stimulator/recorder at said proximate end, characterized by said elongate body being flexible and being stiff enough to be inserted into the external ear canal but flexible enough to bend if said electrode is inserted against the tympanic membrane without rupturing the tympanic membrane, whereby said electrode may be placed in the external ear canal with said distal end being proximate the tympanic membrane or tympano-metal annulus so that the neural/neuromuscular system may be electrically stimulated/recorded.

In an alternative embodiment, an anesthetic may be carried by the compressible material. In other alternative embodiments, a physical fixation device is utilized for physically securing the electrode with respect to the external ear canal. In alternative embodiments, the physical fixation device may be an inflatable cuff, radial fins, a resilient collar or a coiled spring. In an alternative embodiment, adhesive tape adhesively couples the elongated flexible body to the body of the person which in another embodiment may be utilized as a return/reference electrode. In a still further alternative embodiment, the compressible material may be connected to the distal end of the flexible body by a coiled spring.

Brief description of drawings

The foregoing advantages, construction and operation of the present invention will become more readily apparent from the following description and accompanying drawings in which:

Figure 1 is an illustration of an electrode of a preferred embodiment of the present invention;

Figure 2 is an illustration of a method of stimulating the neural/neuromuscular system of a person;

Figure 3 is an illustration of an alternative method to Figure 2;

Figure 4 is an illustration of an electrode of the present invention inserted in an external ear canal;

Figure 5 is an illustration of an alternative embodiment of an electrode of the present invention with an inflatable cuff;

Figure 6 is an illustration of an alternative embodiment of an electrode of the present invention with an inflatable ring;

Figure 7 is an illustration of an alternative embodiment of an electrode of the present invention with a collar;

Figure 8 is an illustration of an alternative embodiment of an electrode of the present invention with fins;

Figure 9 is an illustration of an alternative embodiment of an electrode of the present invention with a speculum and an anesthetic delivery tube; and

Figure 10 is an illustration of an alternative embodiment of an electrode of the present invention with a spring tip.

Detailed description of the preferred embodiments

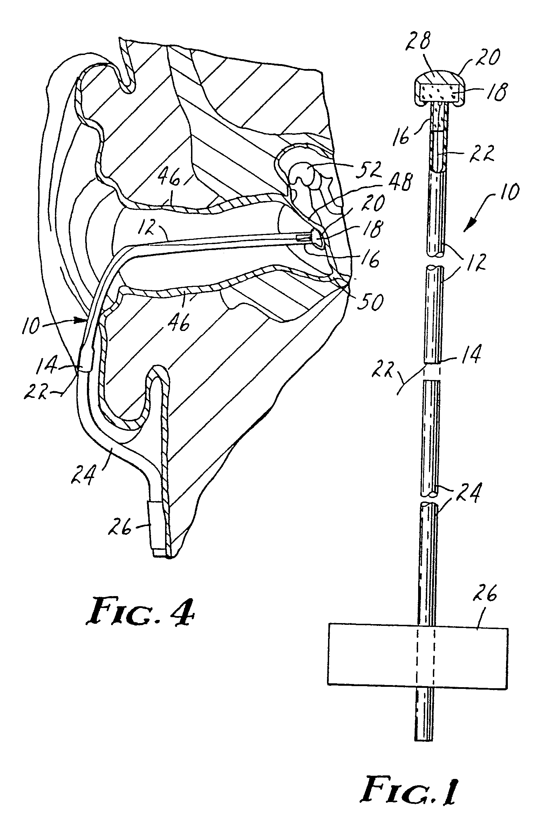

A preferred embodiment of the electrode 10 of the present invention is illustrated in Figure 1. The electrode 10 is formed from a length of silicone elastomer tubing 12 forming an elongated flexible body. The silicone elastomer tubing 12 is of a sufficient size and sufficient stiffness to allow the insertion of the electrode 10 into the external ear canal but is flexible enough so that the silicone elastomer tubing 12 will bend and not create damage to the tympanic membrane should the electrode 10 be inserted in position proximate to tympanic membrane or tympano-meatal annulus. An example of tubing 12 which is suitable for this purpose is Dow Corning Medical Grade HP Silastic™ tubing with an outside diameter of 0.08 inches (2.03 millimeters) and an inside diameter of 0.077 inches (1.96 millimeters). The tubing 12 has a proximate end 14 suitable to be grasped by the insertion means, such as the health care technician's fingers, and a distal end 16 to be placed into the external ear canal. A compressible material 18 is mounted at the distal end 16 of silicone elastomer tubing 12. In a preferred embodiment, the compressible material is a sponge material such as an open cell compressible foam such as a polyester/polyether sponge and is formed to fit snuggly within the distal end 16 of silicone elastomer tubing 12. In a preferred embodiment, the compressible material 18 has an overall diameter larger than the diameter of silicone elastomer tubing 12. In a preferred embodiment, the dimensions of the compressible material 18 are about 0.25 inch (6.4 millimeters) in diameter and about 0.125 inch (3.2 millimeters) in compressible distance. It is preferred that the compressible material also be resilient. The compressible material 18 cushions the tympanic membrane or tympano-meatal annulus when the electrode 10 is inserted approximately at that position. The resiliency in compressible material 18 will allow the electrode 10 to be withdrawn and reinserted with similar insertion characteristics on subsequent insertions into the external ear canal. A conductive gel is carried by the compressible material at the distal end 16 of silicone elastomer tubing 12. A preferred conductive gel is Red Dot™ No. 2248 Solid Conductive Electrode Gel available from Minnesota Mining and Manufacturing Company. An electrical conductor, namely a wire, 22 is inserted through silicone elastomer tubing 12 from the proximate end 14 to the distal end 16 communicating with the conductive gel 20 carried by compressible material 18. Electrical conductor 22 provides the mechanism for supplying the electrical stimulus signal to the distal end 16 of the electrode 10 or to conduct electrical signals generated by the cochlea from the conductive gel 20 to the proximate end 14 of the electrode 10. It is important to electrically insulate electrical conductor 22 from the walls of the external ear canal and is covered with insulation such as Teflon™ insulation. In the preferred embodiment, electrical conductor 22 is carried in the interior of silicone elastomer tubing 12. In other embodiments, it is envisioned that the electrical conductor could be positioned outside of silicone elastomer tubing 12, spirally wrapped or otherwise communicating with silicone elastomer tubing 12 to create a conductive path between the conductive gel 20 at the distal end 16 and the proximate end 14 of electrode 10. A preferred electrical conductor 22 is a 0.003 inch (0.08 millimeters) diameter wire of 90% platinum and 10% iridium with Teflon™ insulation.

Those features of Figure 1 heretofor described contain all the essential features of the electrode 10. In addition, the preferred electrode 10 illustrated in Figure 1 has a second length of silicone elastomer tubing 24 affixed to the proximate end 14 of silicone elastomer tubing 12. One means of securing the second piece of silicone elastomer tubing 24 to silicone elastomer tubing 12 is to make silicone elastomer tubing 24 of a larger diameter and of such a diameter so that a snug fit is developed when silicone elastomeric tubing 24 is placed over or inside of the proximate end 14 of silicone elastomer tubing 12. In a preferred embodiment, electrical conductor 22 may exit the electrode at this point. An alternative electrode 22 could be carried with silicone elastomer tubing 24 the entire length of the electrode or exit somewhere in between. The flexibility characteristics of silicone elastomer tubing 12 are essential for the proper placement of the electrode 10 within the external ear canal. In a preferred embodiment, silicone elastomer tubing 24 is stiffer than and is less flexible than silicone elastomer tubing 12. This silicone elastomer tubing 12 may carry an adhesive tape 26 which can be used to secure the electrode 10 to the external ear, cheek or face of the user providing some mechanical stability to the electrode 10 when it is in place. Any commonly utilized adhesive tape 26 may be utilized such as Micropore™ or Duropore™ tape available from Minnesota Mining and Manufacturing Company.

An electrode constructed as described in Figure 1 affords several advantages over existing technology. The tip, or distal end 16, of the electrode 10 is composed of a compressible material 18, e.g., a sponge, which is infiltrated with a conductive gel 20. The main body of the electrode 10 is a soft silicone elastomer tubing 12 which allows the insertion of the electrode 10 into the external ear canal. The flexibility of the silicone elastomer tubing 12 combined with the compressible material 18 and conductive gel 20 allows the electrode 10 to be positioned proximate the tympanic membrane or tympano-meatal annulus with little or no discomfort. The contact area of the conductive gel 20 with the tympanic membrane or tympano-meatal annulus results in a low impedance connection for recording or stimulating. It has been found that recording with an electrode of this design results in signals which are significantly greater in magnetic than those obtained with a bare conductive ball electrode and provide a more faithful representation of the electrical signals generated by the cochlea. Recording of inner ear potentials is useful in the clinical diagnosis of ear disorders. Further, electrical stimulation of the inner ear via this electrode 10 could be applied for prosthetic, i.e., hearing augmentation, therapeutic, i.e., tinnitus suppression, treatment of hydrops or other inner ear disorders, or diagnostic, i.e. testing of cochlear implant candidates, purposes. The design of the electrode 10 of the present invention affords that current flow into the cochlea is maximized when current is applied directly to the tympanic membrane or tympano-meatal annulus. Less current is lost into the surrounding tissues resulting in (a) lower thresholds for auditory stimulation and (b) less stimulation of cutaneous pain fibers.

Several variations of the electrode 10 could be made without departing from the scope of the present invention. An anesthetic 28 could be incorporated in the compressible material 18. This anesthetic would provide local anesthesia to the stimulus site to further reduce any discomfort from the stimulation or placement of electrode 10 proximate the tympanic membrane or tympano-meatal annulus. A preferred anesthetic to be utilized is lidocaine. Further, it is contemplated that the size of the electrode tip at the distal end 16 could be varied depending upon the optimal use of the electrode, as for example, for stimulation or for recording. For stimulation purposes, a relatively large tip would be optimal since lower current densities at the point of contact would be obtained. A relatively smaller tip at the distal end 16 of the electrode 10 would be optimal for recording purposes where the tip of the electrode 10 would not load or impede the movement of the tympanic membrane.

To insert the electrode 10 into the external ear canal, an insertion tool, typically the health care technician's fingers, grasps the silicone elastomeric tubing 12 and inserts the distal end 16 into the ear canal. The electrode 10 which contains the conductive gel 20, resilient material 18 and, optionally, an anesthetic, if desired, is guided down the external ear canal until the conductive gel 20 of the electrode 10 contacts the tympanic membrane or tympano-meatal annulus. The electrode 10 may then be held in place or may be secured by appropriate physical fixation means, as for example, adhesive tape 26. Electrical conductor 22 may then be connected to the appropriate instrument for either stimulating the inner ear or recording of signals from the inner ear.

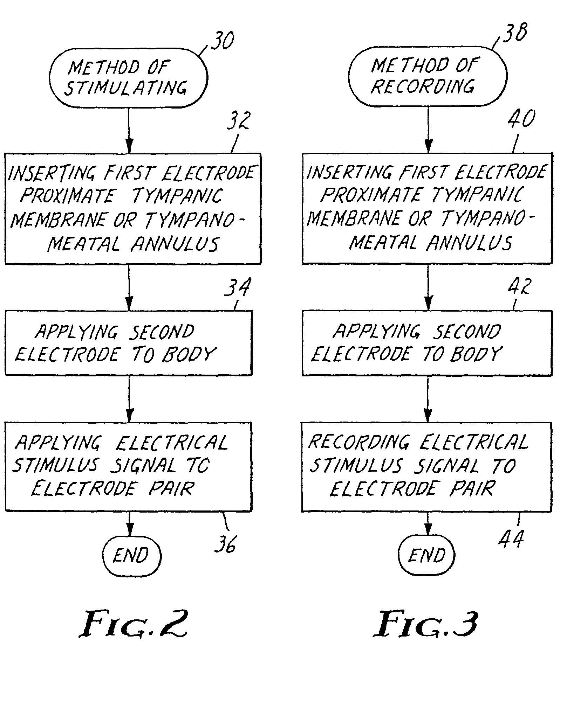

Figure 2 illustrates a method 30 of stimulating the neural/neuromuscular system of a person having a body and an external ear canal and an adjacent tympanic membrane. A first electrode is inserted 32 proximate the tympanic membrane or tympano-meatal annulus. In order to properly localize the current density characteristics and to restrain the electrical current to the appropriate conductive path and to minimize uncomfortableness or pain sensation within the external ear canal, the first electrode should have an elongated flexible body with a proximate end and a distal end. A compressible material is mounted at the distal end of the body. A conductive gel 20 is carried by the compressible material and an electrical conductor communicates with the elongated flexible body and is electrically coupled to the electrically conductive gel. A second electrode is then applied 34 to the body. It is not necessary that the second electrode be of any particular design or that it be applied at any particular location on the body. In general, conventional cutaneous electrodes are applied to the scalp of a person or perhaps to the cheek. The second electrode is a return electrode. An electrical stimulus signal is then applied 36 to the electrode pair consisting of the first electrode inserted in the external ear canal proximate the tympanic membrane or tympano-meatal annulus and the second electrode applied elsewhere to the body.

Similarly, Figure 3 illustrates a method of recording 38 electrical signals from the neural/neuromuscular system of a person having a body and an external ear canal and adjacent tympanic membrane. A first electrode is inserted 40 into the external ear canal proximate the tympanic membrane or tympano-meatal annulus. A first electrode similar to the electrode described with respect to step 32 of Figure 2 is appropriate. As in step 34 of Figure 2, a second electrode is then applied 42 to the body of a person to serve as reference electrode. Similarly, this electrode needs to be of no special design and maybe a conventional cutaneous electrode applied to the scalp, cheek or other portion of the body. The electrical conductors from the electrode pair consisting of the first electrode and the second electrode are then connected to a recording apparatus and the electrical signals obtained from the inner ear are recorded 44.

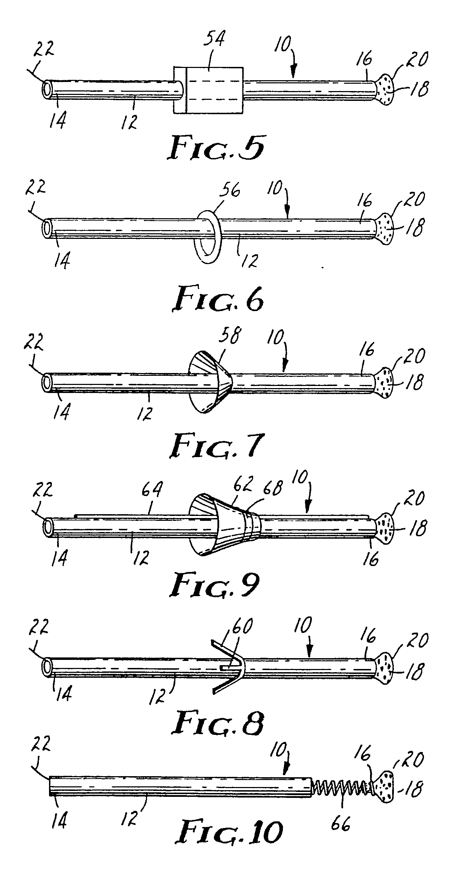

Figure 4 shows the electrode 10 of the present invention inserted into the external ear canal 46 of a patient. In particular, the electrode 10 shown in Figure 1 is illustrated. Silicone elastomer tubing 12 is still enough to be guided into the external ear canal but flexible enough to bend once contact is made with the tympanic membrane 48. Alternatively, the electrode 10 could contact the tympano-meatal annulus 50. The conductive gel 20 of the electrode 10 contacts the tympanic membrane 48 or the tympano-meatal annulus 50. Compressible material 18 is compressed by the contact and positioning of the electrode 10 proximate the tympanic membrane 48. Silastic tubing 24 continues outside the external ear canal 46 and is secured to the cheek of the patient by adhesive tape 26. Electrical conductor 22 may then be connected to the appropriate stimulating or recording source or facility.

It can thus be seen, in reference to Figure 4, that the electrode 10 of the present invention, provides a relatively localized source of stimulation and recording at the tympanic membrane 48 or tympano-meatal annulus 50. Thus, the electrode 10 may take advantage of the most direct current pathway via the ossicular chain 52 to or from the inner ear. The flexibility of the silicone elastomer tubing and the compressibility of the compressible material 18 contribute to the avoidance of pain due to the proximity of the electrode 10 to the tympanic membrane 48 or tympano-meatal annulus 50. Also contributing to the avoidance of pain is the localization of the currents such that stimulations of cutaneous nerve endings in the external ear canal 46 are minimized.

Once the electrode 10 has been placed in the external ear canal 46, it is advantageous to be able to physically fix the electrode 10 with respect to the external ear canal 46. Such fixation will allow consistency of stimulating or recording position as well as prevent damage to the tympanic membrane 48 due to movement of the electrode 10. Several alternative mechanical fixation techniques are described below.

In one alternative embodiment of the electrode 10, the adhesive tape 26 may be made conductive in the form of a cutaneous electrode and the adhesive tape 26 may form the second electrode described in the method of stimulating and the method of recording at steps 34 and 42, respectively, in Figures 2 and 3, respectively.

Figure 5 illustrates an alternative embodiment of an electrode 10. Again, the electrode 10 is formed with an elongated flexible body of a silicone elastomer tubing 12. A compressible material 18 is carried at the distal end 16 of the electrode 10. The compressible material contains a conductive gel 20. An electrical conductor 22 electrically communicates between the end of the electrode 10. Physical fixation of this electrode 10 is achieved through an inflatable cuff 54. Inflatable cuff 54 is preferably a cylindrical balloon constructed from silicone elastomer, preferably 7 mils (0.18 millimeters) thick, fashioned in a manner similar to that of an endotrachial tube and is affixed to silicone elastomer tubing 12. The electrode 10 may be inserted into the external ear canal with the inflatable cuff 54 in a deflated or relatively deflated condition thus allowing insertion of the electrode. Once the electrode has been placed proximate the tympanic membrane or tympano-meatal annulus, the inflatable cuff 54 may be inflated through the insertion of air into its body with a syringe. If removal of the electrode 10 or thereafter desired, the inflatable cuff 54 could be deflated through the reverse process.

Figure 6 illustrates an electrode 10 similarly constructed as the electrode 10 in Figure 5. The electrode 10 in Figure 6 contains as a mechanical fixation means an inflatable ring 56. Inflatable ring 56 may be constructed out of silicone elastomer material. Operating similarly to the inflatable cuff 54 of the electrode 10 of Figure 5, inflatable ring 56 may be in a relatively deflated condition prior to the insertion of the electrode 10 into the external ear canal. Again, once the electrode 10 is inserted into the external ear canal, inflatable ring 56 may be inflated through conventional inflation techniques such as, for example, the addition to the inflatable ring 56 of air via a syringe. Inflatable ring 56 maybe deflated through the reverse process. One advantage of the inflatable ring 56 over the inflatable cuff 54 is that the external ear canal is not occluded when the electrode 10 is in place. The lack of occlusion of the external ear canal may be advantageous in those situations where the electrode 10 has been utilized for recording purposes. Thus, the tympanic membrane 48 could still receive conventional auditory waveforms through the external ear canal without the external ear canal being obstructed.

The electrode 10 illustrated in Figure 7 contains the silicone elastomeric tubing 12 and compressible material 18 and conductive gel 20 at distal end 16 as well as electrical conductor 22. The electrode 10 of Figure 7 for mechanical fixation carries a cone-shaped collar 58 affixed to silicone elastomeric tubing 12. The collar 58 may be constructed from a transparent silicone elastomeric material which would afford visability through the external ear canal by the technician while the electrode 10 is being inserted. The narrow end of the cone of the collar 58 is toward the distal end 16 of the electrode 10. Thus, the electrode 10 would be easily inserted into the ear canal and the collar 58 would take the form of the external ear canal and mechanically fix and hold the electrode 10 in place.

The electrode 10 illustrated in Figure 8 is similar. Again, silicone elastomeric tubing 12 carries electrical conductor 22 to the distal end 16 where the compressible material and conductive gel are located. For mechanical fixation, the electrode 10 carries stabilizing fins 60 affixed to the silicone elastomeric tubing 12. These fins 60 may be constructed of the same silicone elastomeric material as silicone elastomer tubing 12. The fins are affixed to the silicone elastomer tubing 12 and radiate outward and back away from distal end 16 of electrode 10. Thus, the fins 60 perform a springlike effect when the electrode 10 is inserted into the external ear canal and serve to mechanically hold it in place. Since the fins 60 do not completely transversely surround silicone elastomer tubing 12, the external ear canal is not occluded and the health care technician may visually observe the tympanic membrane while the electrode 10 is being inserted and the external ear canal has left unoccluded following insertion.

The electrode 10 illustrated in Figure 9 shows the silicone elastomer tubing 12 being utilized in conjunction with a conventional speculum 62. Again, electrical conductor 22 communicates with silicone elastomer tubing 12 to the compressible material and conductive gel 20 at distal end 16 of the electrode 10. Speculum 62 is of conventional speculum design. The speculum is slideably attached to the silicone elastomer tubing 12 at its narrowest opening. The advantages in the use of speculum 62 with the electrode 10 are that conventional ear health care specialists are very familiar with the speculum and could use it as a guide in placing the electrode proximate the tympanic membrane or the tympano-meatal annulus. Again, the interior of the speculum 62 may be utilized by the health care specialist to visualize the external ear canal and tympanic membrane during the insertion of the electrode 10 into the external ear canal 46. An optional feature of the electrode 10 illustrated in Figure 9 is the use of auxiliary tube 64. Auxiliary tube 64 is coupled longitudinally to silicone elastomer tubing 12 may be utilized either in conjunction with speculum 62 or separately from speculum 62. Through the use of auxiliary tube 64 once the electrode 10 is placed in the external ear canal proximate the tympanic membrane or tympano-meatal annulus an anesthetic may be delivered through the auxiliary tube 64 to the stimulation site of the electrode 10, thus, creating a locally anesthesized area thereby permitting the attainment of higher current densities at that location. It is to be recognized, of course, that auxiliary tube 64 is shown in Figure 9 for illustrative purposes and auxiliary tubes 64 could take many other forms and shapes within the scope of the present invention. Auxiliary tube 64 could alternatively be located within the interior of silicone elastomeric tubing 12 or could take such other forms as would facilitate the delivery of an anesthetic to the distal end 16 of the electrode once the electrode 10 has been inserted into the external ear canal. In one embodiment speculum 62 has a conductive band 68 on the exterior of the speculum 62 to serve as a return/reference electrode.

Figure 10 illustrates an alternative embodiment of the electrode 10 of the present invention. Again, silicone elastomer tubing 12 comprises the elongated flexible body of the electrode 10. Again, electrical conductor 22 communicates with silicone elastomer tubing 12 to a compressible material 18 and a conductive gel 20 at the distal end 16 of the electrode 10. As an additional cushioning means in order to increase the amount of comfort given by the electrode 10 of the present invention a coiled spring 66 is provided between the compressible material 18 and the silicone elastomer tubing 12 at the distal end 16 of the electrode 10. As the compressible material compresses the tympanic membrane 48 coiled spring 66 would contract giving additional flexibility to the electrode 10 and in addition to that flexibility achieved by the compressible material 18 and silicone elastomer tubing 12.

## Claims

1. An electrode (10) adapted to be utilized within the external ear canal (46) for applying/recording electrical signals to/from the neural/neuromuscular system of a person having an external ear canal (46) and adjacent tympanic membrane (48), having an elongate body (12) having a proximate end (14) and a distal end (16), having a compressible material (18) mounted at said distal end (16) of said body (12), having an electrically conductive gel (20) carried by said compressible material (18), and having an electrical conductor (22) coming into contact with said body (12), said electrical conductor (22) being electrically coupled to said electrically conductive gel (20) and adapted to be coupled to a stimulator/recorder at said proximate end (14), characterized by said elongate body (12) being flexible and being stiff enough to be inserted into the external ear canal (46) but flexible enough to bend if said electrode (10) is inserted against the tympanic membrane (48) without rupturing the tympanic membrane (48), whereby said electrode (10) may be placed in the external ear canal (46) with said distal end (16) being proximate the tympanic membrane (48) or tympano-meatal annulus (50) so that the neural/neuromuscular system may be electrically stimulated/recorded.

2. An electrode (10) as in claim 1 wherein said compressible material (18) is absorbent and said electrically conductive gel (20) is abosrbed into said compressible material (18).

3. An electrode (10) as in claim 2 wherein said compressible material (18) is also resilient.

4. An electrode (10) as in claim 1 which is further characterized by having an anesthetic (28) carried by said compressible material (18).

5. An electrode (10) as in claim 1 which is further characterized by having a fixation means (26) for physically securing said electrode (10) with respect to the external ear canal (46), said fixation means (26) being coupled to said body (12).

6. An electrode (10) as in claim 1 wherein said compressible material (18) comprises a sponge.

7. An electrode (10) as in claim 1 wherein said body (12) comprises silicone elastomeric tubing.

8. An electrode (10) as in claim 1 wherein said compressible material (18) is connected to said distal end (16) of said silicone elastomeric tubing by a coiled spring (66).

9. An electrode (10) as in claim 8 wherein said coiled spring (66) comprises said electrical conductor (22).

10. An electrode (10) as in claim 1 wherein said elongated body (12) is electrically conductive and serves as said electrical conductor (22).

11. An electrode (10) as in claim 1 wherein said electrical conductor (22) has a resistance of not more than about ten kilohms.

12. An electrode (10) as in claim 1 wherein said body has a first portion (12) which is more flexible than a second portion (24) of said body, said second portion (24) of said body being closer to said proximate end (14) than said first portion (12).

**Patentansprüche**

1. Elektrode (10), die in dem äußeren Gehörgang (46) zum Anlegen/Aufnehmen elektrischer Signale an das/von dem Neuralsystem/Neuromuskularsystem einer Person verwendbar ist, die einen äußeren Gehörgang (46) und ein diesem benachbartes Trommelfell (48) besitzt, mit einem langgestreckten Körper (12), der ein proximales Ende (14) und ein distales Ende (16) besitzt und an dessen distalem Ende ein kompressibles Material (18) angebracht ist, das ein elektrisch leitfähiges Gel (20) trägt, und mit einem mit dem Körper (12) in Berührung kommenden elektrischen Leiter (22), der mit dem elektrisch leitfähigen Gel (20) elektrisch gekuppelt und mit einem an dem proximalen Ende (14) vorgesehenen Stimulator/Aufnehmer kuppelbar ist, dadurch gekennzeichnet, daß der langgestreckte Körper (12) flexibel und so steif ist, daß er in den äußeren Gehörgang (46) einführbar ist, aber so flexible, daß er gebogen wird, wenn die Elektrode (10) gegen das Trommelfell (48) eingeführt wird, ohne dieses zu zerreißen, so daß die Elektrode (10) in dem äußeren Gehörgang (46) derart angeordnet werden kann, daß sich das distale Ende (16) in der Nähe des Trommelfells (48) oder des von dem Trommelfell und dem Gehörgang gebildeten Ringkörpers (50) befindet, so daß das Neuralsystem/Neuromuskularsystem elektrisch angeregt/überwacht werden kann.

2. Elektrode (10) nach Anspruch 1, dadurch gekennzeichnet, daß das kompressible Material (18) absorptionsfähig ist und daß das elektrisch leitfähige Gel (20) von dem kompressiblen Material (18) absorbiert ist.

3. Elektrode (10) nach Anspruch 2, dadurch gekennzeichnet, daß das kompressible Material (18) auch elastisch ist.

4. Elektrode (10) nach Anspruch 1, dadurch gekennzeichnet, daß das kompressible Material (18) ein Anästhetikum (28) trägt.

5. Elektrode (10) nach Anspruch 1, dadurch gekennzeichnet, daß mit dem Körper (12) eine Befestigungseinrichtung (26) zum physischen Befestigen der Elektrode (10) relativ zu dem äußeren Gehörgang (46) gekuppelt ist.

6. Elektrode (10) nach Anspruch 1, dadurch gekennzeichnet, daß das kompressible Material (18) wenigstens teilweise aus einem Schwamm besteht.

7. Elektrode (10) nach Anspruch 1, dadurch gekennzeichnet, daß der Körper (12) wenigstens teilweise aus elastomerem Schlauchmaterial aus Silicon besteht.

8. Elektrode nach Anspruch 1, dadurch gekennzeichnet, daß das kompressible Material (18) mit dem distalen Ende (16) des elastomeren Schlauchmaterials aus Silicon durch eine Schraubenfeder (66) verbunden ist.

9. Elektrode (10) nach Anspruch 8, dadurch gekennzeichnet, daß die Schraubenfeder (66) wenigstens teilweise aus dem elektrischen Leiter (22) besteht.

10. Elektrode (10) nach Anspruch 1, dadurch gekennzeichnet, daß der langgestreckte Körper (12) elektrisch leitfähig ist und als der genannte elektrische Leiter (22) dient.

11. Elektrode (10) nach Anspruch 1, dadurch gekennzeichnet, daß der elektrische Leiter (22) einen Widerstand von nicht mehr als etwa 10 Kiloohm hat.

12. Elektrode (10) nach Anspruch 1, dadurch gekennzeichnet, daß das Körper einen ersten Teil (12) besitzt, der flexibler ist als ein zweiter Teil (24) des genannten Körpers, und daß der zweite Teil (24) des genannten Körpers dem proximalen Ende (14) näher ist als der erste Teil (12).

**Revendications**

1. Electrode (10) adaptée pour être utilisée dans le conduit auditif externe (46) pour appliquer des signaux électriques au système nerveux ou neuromusculaire ou enregistrer des signaux électriques provenant de ce système chez une personne possédant un conduit auditif externe (46) et une membrane de tympan adjacente (48), ladite électrode comportant un corps allongé (12) présentant une extrémité proximale (14) et une extrémité distale (16), un matériau compressible (18) monté sur ladite extrémité distale (16) dudit corps (12), un gel électroconducteur (20) porté par ledit matériau compressible (18), un conducteur électrique (22) venant au contact dudit corps (12), ledit conducteur électrique (22) étant couplé électriquement audit gel électroconducteur (20) et adapté pour être couplé à un stimulateur ou un enregistreur à ladite extrémité proximale (14), caractérisée en ce que ledit corps allongé (12) est flexible et suffisamment rigide pour être inséré dans le conduit auditif externe (46) mais suffisamment flexible pour fléchir si ladite électrode (10) bute contre la membrane de tympan (48) sans déchirer la membrane de tympan (48), moyennant quoi on peut placer ladite électrode (10) dans le conduit auditif externe (46) avec ladite extrémité distale (16) à proximité de la membrane de tympan (48) ou de l'anneau tympanal (50) de sorte que le système nerveux ou neuromusculaire puisse être électriquement stimulé ou enregistré.

2. Electrode selon la revendication 1, dans laquelle ledit matériau compressible (18) est absorbant et ledit gel électroconducteur (20) est absorbé dans ledit matériau compressible (18).

3. Electrode selon la revendication 2, dans laquelle ledit matériau compressible (18) est également élastique.

4. Electrode selon la revendication 1, caractérisée en outre par un anesthésique (28) porté par ledit matériau compressible (18).

5. Electrode selon la revendication 1, caractérisée en outre par des moyens de fixation (26) pour fixer physiquement ladite électrode (10) par rapport au conduit auditif externe (46), lesdits moyens de fixation étant couplés audit corps (12).

6. Electrode selon la revendication 1, dans laquelle le matériau compressible (18) comprend un matériau spongieux.

7. Electrode selon la revendication 1, dans laquelle ledit corps comprend un tubage en silicone élastomère.

8. Electrode selon la revendication 1, dans laquelle ledit matériau compressible (18) est relié à ladite extrémité distale (16) du tubage en silicone élastomère par un ressort hélicoïdal (66).

9. Electrode selon la revendication 8, dans laquelle ledit ressort hélicoïdal (66) comprend ledit conducteur électrique (22).

10. Electrode selon la revendication 1, dans laquelle ledit corps allongé (12) est électroconducteur et constitue ledit conducteur électrique (22).

11. Electrode selon la revendication 1, dans laquelle ledit conducteur électrique (22) a une résistance ne dépassant pas environ 10 kilohms.

12. Electrode selon la revendication 1, dans laquelle ledit corps présente une première partie (12) qui est plus flexible qu'une seconde partie (24) dudit corps, ladite seconde partie (24) dudit corps étant plus proche de ladite extrémité proximale (14) que ladite première partie (12).

FIG. 4

FIG. 1

1

```
                  ╭──────────────╮ 30              ╭──────────────╮ 38
                  │  METHOD OF   │                 │  METHOD OF   │
                  │  STIMULATING │                 │  RECORDING   │
                  ╰──────────────╯                 ╰──────────────╯
                         │          32                    │          40
                         ▼                                ▼
        ┌─────────────────────────────┐  ┌─────────────────────────────┐
        │ INSERTING FIRST ELECTRODE   │  │ INSERTING FIRST ELECTRODE   │
        │ PROXIMATE  TYMPANIC         │  │ PROXIMATE TYMPANIC          │
        │ MEMBRANE OR TYMPANO-        │  │ MEMBRANE OR TYMPANO-        │
        │ MEATAL ANNULUS              │  │ MEATAL ANNULUS              │
        └─────────────────────────────┘  └─────────────────────────────┘
                         │          34                    │          42
                         ▼                                ▼
        ┌─────────────────────────────┐  ┌─────────────────────────────┐
        │  APPLYING  SECOND           │  │  APPLYING  SECOND           │
        │  ELECTRODE  TO BODY         │  │  ELECTRODE  TO BODY         │
        └─────────────────────────────┘  └─────────────────────────────┘
                         │                                │
                         ▼                                ▼
        ┌─────────────────────────────┐  ┌─────────────────────────────┐
        │ APPLYING  ELECTRICAL        │  │ RECORDING ELECTRICAL        │
        │ STIMULUS SIGNAL TC          │  │ STIMULUS SIGNAL TO          │
        │ ELECTRODE PAIR              │  │ ELECTRODE PAIR              │
        └─────────────────────────────┘  └─────────────────────────────┘
                         │                                │
                         ▼          36                    ▼          44
                     ╭───────╮                        ╭───────╮
                     │  END  │                        │  END  │
                     ╰───────╯                        ╰───────╯
```

$$FIG.2 \qquad\qquad FIG.3$$

FIG. 5

FIG. 6

FIG. 7

FIG. 9

FIG. 8

FIG. 10